# EUROPEAN PATENT SPECIFICATION

(11) **EP 1 738 678 B1**
(45) Date of publication and mention of the grant of the patent: **04.05.2016**
(21) Application number: 05719839.2
(22) Date of filing: 02.03.2005
(51) Int. Cl.: A61B 1/00, F16H 1/46

(54) **ENDOSCOPE**
ENDOSKOP
ENDOSCOPE

(30) Priority: 10.03.2004 JP 2004067903
(43) Date of publication of application: 03.01.2007
(73) Proprietor: Olympus Corporation, Tokyo 151-0072 (JP)
(72) Inventor: UENO, Haruhiko;c/o Olympus Med. Systems Corp., Tokyo 151-0072 (JP); MASAKI, Yutaka, 1810012 (JP); SAITO, Shoichi, 1930824 (JP)
(74) Representative: von Hellfeld, Axel
(86) International application number: PCT/JP2005/003524
(87) International publication number: WO 2005/087081

(56) References cited:
- WO-A-00/63588
- JP-A- 2000 274 495
- JP-A- 2002 125 919
- US-A- 5 060 632

## Description

### TECHNICAL FIELD

The present invention relates to an endoscope that bends a bendable portion provided at an insertion portion by pulling and loosening a traction member extending from the bendable portion by driving a driving motor of a bending manipulating device.

### BACKGROUND ART

Conventionally, an endoscope is widely used in a medical field and an industrial field. In a general endoscope, a bendable portion that can be bent, for example, in upward and downward directions and in leftward and rightward directions, is provided at an elongated insertion portion. The bendable portion is made to bend when a traction member such as an angle wire that runs through inside the insertion portion is pulled and/or loosened through a manipulation of a manipulating lever provided in a manipulating portion.

Recently, an endoscope that is provided with an electric bending manipulating device in, for example, a manipulating portion thereof has been used to save power for a bending manipulation of the bendable portion. In the endoscope that is provided with the electric bending manipulating device, when a controller such as a joystick is tilted, a driving force of an electric motor causes pulling and loosening of a predetermined traction member. As a result, the bendable portion is bent.

For example, a bending manipulating device of an endoscope described in Japanese Patent No. 2660053 transmits rotational driving force of a motor disposed inside a manipulating portion to a gear on a driving side through a train of bevel gears. Then, torque of the gear on the driving side is transmitted to a gear on a driven side while a speed of the gear on the driving side is reduced. Since a sprocket is integrated with the gear on the driven side, the sprocket is rotated when the gear on the driven side is rotated. Consequently, a chain geared with the sprocket is moved, and the bendable portion is bent since one end of a wire that is secured to the chain is pulled and loosened.

Patent document 1:Japanese Patent No. 2660053

US patent application US 5,060,632 A discloses an endoscope with a common reduction speed mechanism, which however comprises additional gears which reduce the power efficiency of the endoscope.

PCT patent application WO 00/63588 A discloses a general purpose planetary reduction speed gearbox which is not disclosed for an endoscope.

### DISCLOSURE OF INVENTION

### PROBLEM TO BE SOLVED BY THE INVENTION

The bending manipulating device of the endoscope described in Japanese Patent No. 2660053 employs a speed reduction mechanism with a few gears. Hence, it is required to use a large gear and a large motor to generate large torque in order to maintain driving torque. However, the motor that generates the large torque consumes a large amount of electric currents and a size of the motor becomes large. Consequently, there is an inconvenience that the bending manipulating device becomes large and heavy. Thus, it is necessary to increase the number of gears for the speed reduction to avoid increase in the size of the motor; however, the increase in the number of gears for the speed reduction results in increase in the size of the bending manipulating device.

Further, the driving force of the motor is transmitted to the gear on the driving side through a part such as a bevel gear where axes of shafts intersect. There is an inconvenience that the gear might be expensive since extremely accurate gearing is required within the gears just mentioned.

The present invention is provided to alleviate the above inconveniences, and an object of the present invention is to provide a simply structured downsizable endoscope that includes a bending manipulating device that can acquire a desired speed reduction ratio and torque.

### MEANS FOR SOLVING PROBLEM

An endoscope of the present invention includes an insertion portion that includes a bendable portion having plural bending pieces connected with each other; and a bending manipulating device that advances and retreats a traction member which extends from the bendable portion by driving force of a driving motor to bend the bendable portion, wherein the bending manipulating device is provided with a rotating member that rotates with the traction member engaged, the driving motor that rotates the rotating member, and a speed reduction mechanism that transmits the driving force of the driving motor to the rotating member, and the speed reduction mechanism includes a sun gear that is rotated by the driving force of the motor and that has external teeth with a predetermined number of teeth, plural planetary gears that includes external teeth which engage with the external teeth of the sun gear, the external teeth of the planetary gears having a predetermined number of teeth, a first gear that is a fixed gear having internal teeth which engage with the external teeth of the planetary gears, the internal teeth of the first gear having a predetermined number of teeth, and a second gear that is a movable gear having internal teeth which engage with the external teeth of the planetary gears, the internal teeth of the second gear having a predetermined number of teeth different from the number of teeth of the internal teeth of the first gear by a predetermined number.

Then, a motor shaft of the driving motor is protruded in a direction orthogonal to a traction direction of the traction member, and the sun gear is provided on the motor shaft.

Further, the second gear has external teeth, and the rotating member further has a transmitting gear which is integrally formed with the rotating member and which includes external teeth formed by a predetermined number of teeth for engaging with the external teeth of the second gear.

Further, the rotating member is formed coaxially with the transmitting gear and disposed away from the driving motor with respect to the transmitting gear, and the second gear is disposed between the driving motor and the first gear.

Furthermore, an outside diameter of the second gear and an outside diameter of the driving motor are substantially the same, or the outside diameter of the second gear is smaller than the outside diameter of the driving motor.

Further, the first gear is rotatably disposed, and the endoscope further includes a switching mechanism that properly switches a state of the first gear to one of a fixed gear state and a movable gear state.

According to the configurations described above, the planetary gears start to rotate and revolve when the sun gear is rotated, since the first gear that is the fixed gear having the internal teeth does not rotate. Since the number of the internal teeth of the first gear whose internal teeth are formed so as to gear with the external teeth of the planetary gears and the number of the internal teeth of the second gear, which is the movable gear, differ by a predetermined number, a geared internal tooth of the first gear is shifted with respect to a geared internal tooth of the second gear. When the planetary gears continue to rotate and revolve, the rotation of the second gear is slowed down so as to compensate for the shifting. Further, the speed reduction mechanism can be manufactured at low cost when the sun gear, the planetary gears, the first gear, and the second gear are spur gears.

Further, the sun gear, the planetary gears, the first gear, and the second gear that configure the speed reduction mechanism are arranged in a superposed manner with respect to the motor. Therefore, a thickness of the manipulating portion can be suppressed by configuring a flat motor and by making a width of a tooth of the gear as thin as possible within a manageable level.

Further, since the motor shaft of the driving motor differs from the rotating shaft of the rotating member, the driving motor and the rotating member are not superposed coaxially. Consequently, a thickness of the speed reduction mechanism in the direction of the motor shaft can be decreased, so that the speed reduction mechanism can be miniaturized. Here, in addition to the configuration in claim 2, the manipulating portion with an optimized balance can be configured by setting the distance between the motor shaft and the rotating shaft in view of the length and the width of the manipulating portion.

Furthermore, the outside diameter of the second gear is made substantially the same as the outside diameter of the motor. Then, a center distance of the motor shaft and the rotating member is decreased. Consequently, the speed reduction mechanism can be downsized.

Further, the switching mechanism switches the state of the first gear, which is rotatably arranged, to the fixed gear state or the movable gear state as necessary. Thus, a state in which the driving force of the motor is transmitted to the rotating member and a state in which the driving force of the motor is not transmitted to the rotating member can be obtained.

### EFFECT OF THE INVENTION

According to an endoscope of the present invention, a simply structured small endoscope having a bending manipulating device that can obtain a desired speed reduction ratio and torque can be provided.

### BRIEF DESCRIPTION OF DRAWINGS

FIG. 1 is a drawing illustrating a configuration of an endoscope device;
FIG. 2 is a top view illustrating a configuration of a bending device;
FIG. 3 is a bottom view illustrating the configuration of the bending device;
FIG. 4 is a view illustrating gears configuring a major portion of a bending manipulating device;
FIG. 5 is a view illustrating a positional relationship of an arrangement and a gearing relationship of the gears of the bending manipulating device; and
FIG. 6 is a view illustrating a major portion of a switching mechanism.

### EXPLANATIONS OF LETTERS OR NUMERALS

20 Bending device
22, 23 Bending manipulating device
24 Motor
30 Speed reduction mechanism
31 Sun gear
32 Planetary gear
33 Fixed gear
34 Movable gear
41 Rotating member
42 Rotating external gear
50 Switching mechanism
51 Clutch lever
52 Clutch cam
53 Clutch lever shaft

### BEST MODE(S) FOR CARRYING OUT THE INVENTION

Exemplary embodiments of the present invention will be explained below with reference to the accompanying drawings. An endoscope in which a bending manipulating device is installed in a manipulating portion is explained as an example in the explanation hereinafter. However, the present invention is not limited to the configuration described above, and, for example, the bending manipulating device and the manipulating portion can be provided separately.

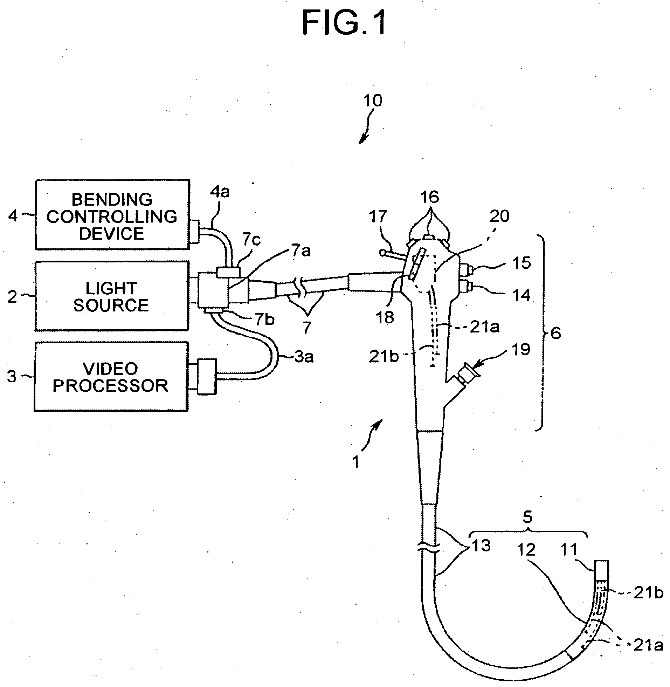

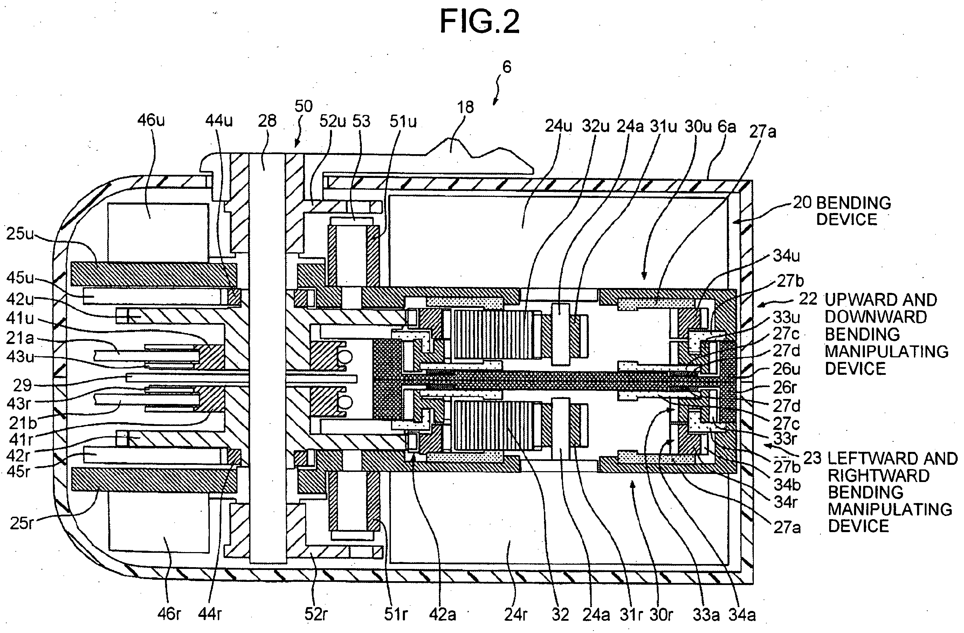

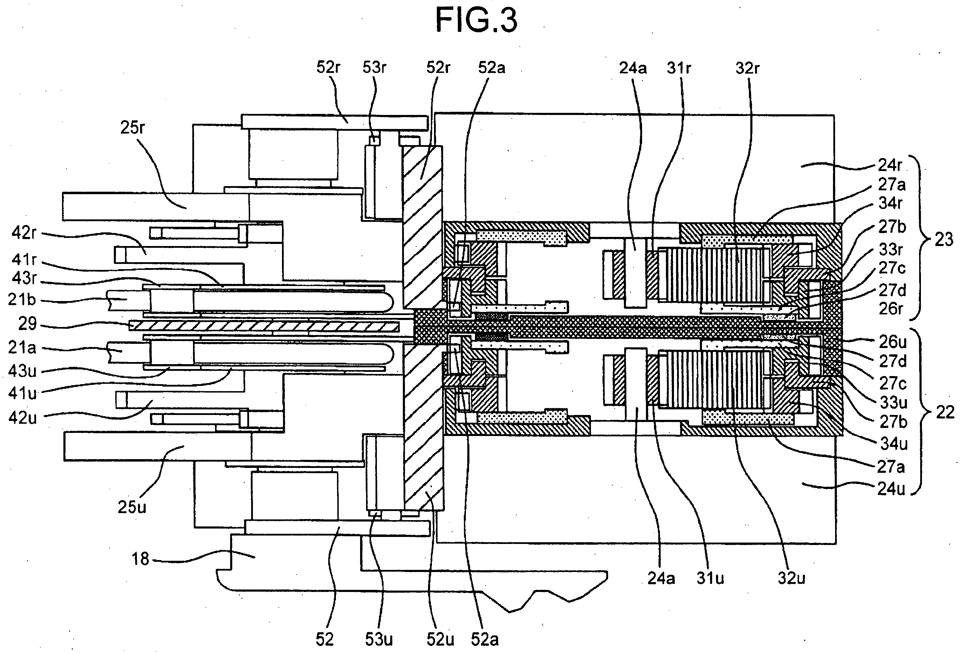

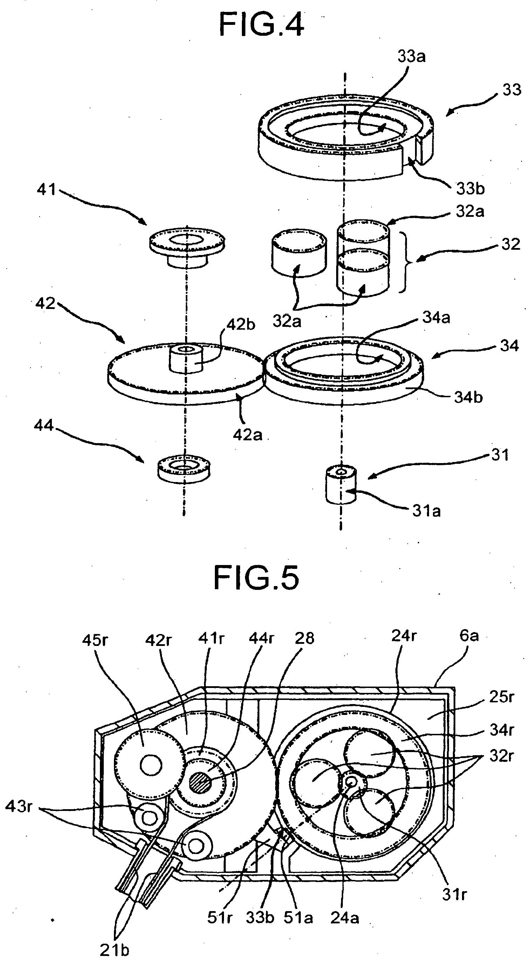

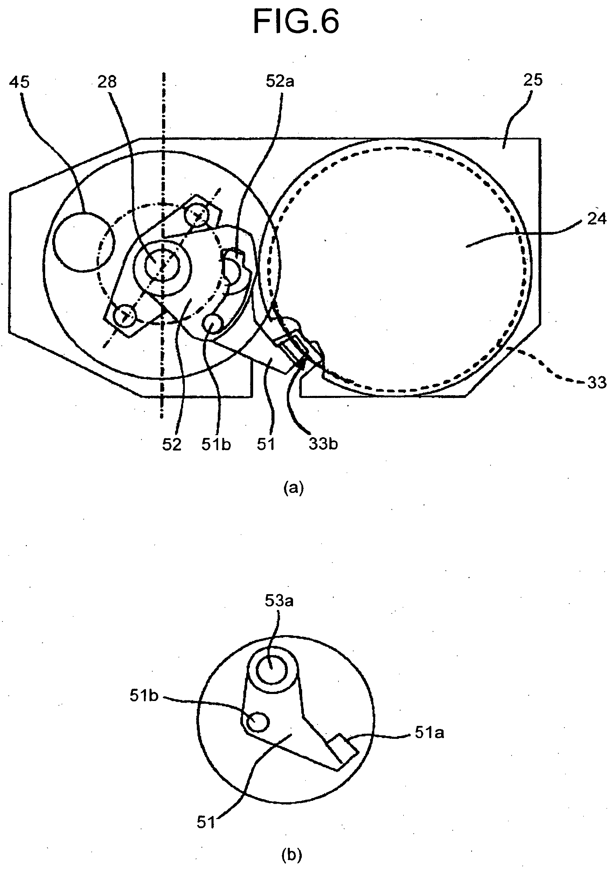

FIGS. 1 to 6 correspond to an embodiment of the present invention. FIG. 1 is a view illustrating a configuration of an endoscope device; FIG. 2 is a top view illustrating a configuration of a.bending device; FIG. 3 is a bottom view illustrating the configuration of the bending device; FIG. 4 is a drawing illustrating gears configuring a major portion of a bending manipulating device; FIG. 5 is a drawing illustrating a positional relationship of an arrangement and a gearing relationship of the gears of the bending manipulating device; and FIG. 6 is a view illustrating a major portion of a switching mechanism.

FIG. 6(a) is a view illustrating a detail of the switching mechanism and a relationship between a clutch lever and a fixed gear, and FIG. 6(b) is a view illustrating the clutch lever.

As shown in FIG. 1, an endoscope device 10 having an endoscope 1 of the present invention mainly includes the endoscope 1; a light source 2 that supplies illumination light to the endoscope 1; a video processor 3 that performs a signal processing for an imaging device (not shown) installed in the endoscope 1; and a bending controlling device 4 that controls driving of a driving motor described later. The driving motor is a part of a later described bending device 20 provided in the endoscope 1.

The endoscope 1 includes an elongated insertion portion 5; a manipulating portion 6 that is provided at a proximal end side of the insertion portion 5 and also functions as a grip; and a universal cord 7 that extends from a side of the manipulating portion 6.

The insertion portion 5 has a rigid distal end portion 11; a bendable portion 12 that is bendable in directions of, for example, leftward and rightward and in directions of upward and downward, and connected to a proximal end side of the distal end portion 11; and a flexible pipe 13 having flexibility and connected to a proximal end side of the bendable portion 12. The distal end portion 11, the bendable portion 12, and the flexible pipe 13 are arranged in this order from the distal end side of the insertion portion 5.

The bendable portion 12 is configured to be bent in the upward and downward directions and in the leftward and rightward directions, by rotatably connecting plural bending pieces not shown to each other. An upward and downward direction manipulating wire 21a and leftward and rightward direction manipulating wire 21b are extended from the top bending piece of the bendable portion 12.

The manipulating portion 6 includes an air and water supply button 14 employed for water and air supply manipulation; an aspiration button 15 employed for aspiration manipulation; plural video switches 16 employed for remote control of the video processor 3; a device such as a joystick 17 outputting a command for pulling and loosening the wires 21a and 21b to bend the bendable portion 12; a manipulating lever 18 that is a part of a switching mechanism 50; and a surgical instrument insertion port 19 into which a surgical instrument such as a bioptome is inserted. The switching mechanism 50 switches between a state where the wires 21a and 21b are pulled due to the driving force of the driving motor provided in the bending device 20 and a state where the wires 21a and 21b are released from the pulling by the driving force of the driving motor. Here, proximal end portions of the wires 21a and 21b are disposed at the bending device 20.

A light source connector 7a that is detachably connected to the light source 2 is provided at an end of the universal cord 7. A video connector portion 7b and a bending controlling connector portion 7c are each provided on a side of the light source connector 7a. Here, a video cable 3a that is electrically connected to the video processor 3 is detachably connected to the video connector portion 7b, and an electric Cable 4a that is electrically connected to the bending controlling device 4 is detachably connected to the bending controlling connector portion 7c.

A configuration and an effect of the bending device 20 are explained with reference to FIGS. 2 to 6.

As shown in FIGS. 2 and 3, the bending device 20 disposed inside an exterior case body 6a of the manipulating portion 6 includes a pair of bending manipulating devices, i.e., an upward and downward bending manipulating device 22 and a leftward and rightward bending manipulating device 23; and the switching mechanism 50.

Configurations and effects of the leftward and rightward bending manipulating device 23 and the upward and downward bending manipulating device 22 are substantially the same. Hence, when the configurations and the effects of the upward and downward bending manipulating device 22 and the leftward and rightward bending manipulating device 23 are explained, the configuration and the effect of the upward and downward bending manipulating device 22 are mainly explained, and the explanation is not to be repeated for the configuration and the effect of the leftward and rightward bending manipulating device 23.

Respective members constituting the upward and downward bending manipulating device 22 and the leftward and rightward bending manipulating device 23 are explained without letters for distinguishing members for upward and downward from members for leftward and rightward. When it is necessary to distinguish the members for the upward and downward bending manipulating device 22 from the members for the leftward and rightward bending manipulation device 23, a letter "u" is affixed to the reference character for each member for the upward and downward bending manipulation device 22, and a letter "r" is affixed for each member for the leftward and rightward bending manipulation device 23. Further, in the drawings, the letter "u" is affixed to the reference character of each member for the upward and downward bending manipulation device 22, and the letter "r" is affixed for each member for the leftward and rightward bending manipulation device 23 in order to distinguish the members for the upward and downward bending manipulation device 22 from the members for the leftward and rightward bending manipulation device 23.

The bending manipulating device 22 mainly includes a driving motor 24 (hereinafter referred to as motor), a speed reduction mechanism 30, and a rotating member 41.

The motor 24 has a flat shape and is secured to an exterior case body 6a side of a flame 25 having a step shape.

A configuration of the speed reduction mechanism 30 is explained in detail with reference to FIGS. 2 to 4.

The speed reduction mechanism 30 mainly includes a sun gear 31 that consists of a spur gear disposed on a motor shaft 24a of the motor 24; three planetary gears 32, 32, and 32 that are, for example, spur gears; a fixed gear 33 that is a first gear and consists of an internal gear; and a movable gear 34 that is a second gear and consists of an internal gear. The sun gear 31, the planetary gears 32, 32, 32, the fixed gear 33, and the movable gear 34 are disposed in a case that consists of the flame 25 and a cap 26.

The movable gear 34 has a substantially ring shape and is disposed at a motor 24 side. The movable gear 34 has internal teeth 34a including a predetermined number of teeth formed on an inner circumferential face side thereof and external teeth 34b including a predetermined number of teeth formed on an outer circumferential face side thereof. The movable gear 34 is disposed between a first thrust receiver 27a and a second thrust receiver 27b so that the movable gear 34 can rotate around the motor shaft 24a as a central axis. The first thrust receiver 27a is securely fixed to the flame 25. The second thrust receiver 27b is disposed so as to be held between the cap 26 and the flame 25.

The fixed gear 33 has a ring shape which is substantially the same as that of the movable gear 34. The fixed gear 33 has internal teeth 33a having a predetermined number of teeth provided on an inner circumferential face side of the fixed gear 33, and an engaging groove 33b having a depressed shape formed on an outer circumferential face side thereof. The number of teeth in the internal teeth 33a of the fixed gear 33 differs from the number of teeth in the internal teeth 34a of the movable gear 34 by a predetermined amount. In the present embodiment, since there are three planetary gears, the difference between the numbers of teeth is three. For example, the number of teeth in the internal teeth 33a of the fixed gear 33 is set to be greater than the number of teeth in the internal teeth 34a of the movable gear 34.

The difference between the number of teeth in the internal teeth 33a of the fixed gear 33 and the number of teeth in the internal teeth 34a of the movable gear 34 is set to four (when there are four planetary gears) or six (when there are six planetary gears) corresponding to torque. Further, a shape of the internal teeth 33a of the fixed gear 33 is projected onto a shape of the internal teeth 34a of the movable gear 34 so that the planetary gear 32 gears with the internal teeth 33a of the fixed gear 33 and with the internal teeth 34a of the movable gear 34 while keeping an identical center distance.

The fixed gear 33 is also disposed between the second thrust receiver 27b and a third thrust receiver 27c so as to rotate around the motor shaft 24a as a central axis. An engaging portion 51a, which is provided on a rotatable clutch lever 51, engages with an engaging groove 33b of the fixed gear 33.

When the endoscope of the present embodiment is used in a normal state, the engaging portion 51a of the clutch lever 51 is engaged with the engaging groove 33b of the fixed gear 33 that is rotatably disposed between the second thrust receiver 27b and the third thrust receiver 27c. Consequently, the rotatably disposed gear functions as the fixed gear 33 as described above.

The third thrust receiver 27c is installed securely on the cap 26. The letter 27d represents an elastic member (spring member in the present embodiment) that is a member biasing the third thrust receiver 27c toward the motor 24. The bendable portion 12 that has been bent is prevented from rapidly coming back to straighten when the engaging portion 51a of the clutch lever 51 that has been engaged with the engaging groove 33b of the fixed gear 33 is disengaged, since the spring member 27d biases the third thrust receiver 27c.

Outside diameters of the fixed gear 33 and the movable gear 34 are substantially identical to or smaller than an outside diameter of the motor 24. Consequently, the device can be downsized by reducing a center distance between the motor shaft 24a and a rotating member supporting shaft 28 described below that is a supporting shaft of the rotating member 41.

External teeth 32a are formed on the planetary gear 32. The external teeth 32a engage with the external teeth of the sun gear 31, as well as gear with the internal teeth 33a of the fixed gear 33 and the internal teeth 34a of the movable gear 34. That is to say, a tooth width of the planetary gear 32 is such that the planetary gear 32 engages with each of the internal teeth 33a of the fixed gear 33 and the internal teeth 34a of the movable gear 34. The planetary gears 32, 32, and 32 are rotatably disposed between the first thrust receiver 27a and the third thrust receiver 27c.

An effect of the speed reduction mechanism 30 that is configured as described above is explained.

When the motor 24 is driven, rotational driving force of the motor 24 is transmitted to the movable gear 34 as described below.

First, the sun gear 31 that is disposed on the motor shaft 24a is rotated when the motor 24 is driven. Next, the rotation of the sun gear 31 is transmitted to the planetary gears 32, 32, and 32. Then, the planetary gears 32, 32, and 32 are started to rotate. At the moment, the engaging portion 51a is engaged with the engaging groove 33b in order not to rotate the fixed gear 33. Consequently, the planetary gears 32, 32, and 32 that are engaged with the fixed gear 33 and the movable gear 34 start to revolve around the sun gear 31 with the rotations thereof. That is to say, the planetary gears 32, 32, and 32 start to rotate and revolve by torque transmitted from the sun gear 31.

Here, the internal teeth 33a of the fixed gear 33 and the internal teeth 34a of the movable gear 34 are both geared with the external teeth 32a of the planetary gears 32, 32, and 32. The number of internal teeth 33a of the fixed gear 33 is different from the number of internal teeth 34a of the movable gear 34 by a predetermined number. Hence, a geared internal tooth 33a of the fixed gear 33 is shifted with respect to an internal tooth 34a of the movable gear 34 that is engaged with the external teeth 32a of the planetary gear 32. During the rotation and revolution of the planetary gears 32, 32, and 32, the movable gear 34 rotates in such a manner as to compensate for the shifting.

The movable gear 34 is continued to rotate with a reduced speed compared to the rotation of the motor 24 by continuously driving the motor 24 and by rotating and revolving the planetary gears 32, 32, and 32.

A configuration around the rotating member 41 is explained with reference to FIGS. 2 to 5.

The wire 21a or the wire 21b is integrally fixed to the rotating member 41. The rotating member 41 is integrally fixed to a large-diameter shaft 42b formed on a rotating external gear 42 that is a transmitting gear having external teeth 42a. The external teeth 42a engages with the external teeth 34b of the movable gear 34. The rotating external gear 42 that is integrated with the rotating member 41 is rotatably disposed with respect to the rotating member supporting shaft 28 in such a way that the rotating external gear 42 is arranged between the flame 25u and a separating board 29.

Consequently, the movable gear 34 and the rotating external gear 42 are prevented from being arranged so as to overlap each other with respect to the motor 24 since a position of the rotating member supporting shaft 28 and a position of the motor shaft 24a of the motor 24 are different. Therefore, a dimension in an extending direction of the motor shaft 24a of the motor 24 is suppressed from becoming large. That is to say, a width (thickness) of the manipulating portion is suppressed from becoming large.

When the motor 24 is continuously driven, the planetary gears 32, 32, and 32 are continued to rotate and revolve. Consequently, the movable gear 34 is rotated, and the rotating external gear 42 having the external teeth 42a that engage with the external teeth 34b of the movable gear 34 is rotated. Then, the rotating member 41 that is integrally secured to the rotating external gear 42 is rotated. Therefore, the wires 21a and 21b that are integrally disposed on the rotating member 41 are pulled and loosened. Each of the wires 21a and 21b that are integrally disposed on the rotating member 41 is held by a guide roller 43 disposed on the separating board 29.

Since the rotating members 41u and 41r that respectively form a part of the upward and downward bending manipulating device 22 and the leftward and rightward bending manipulating device 23 are arranged with the separating board 29 therebetween, the rotating members 41u and 41r do not come into contact with each other, and similarly, the wires 21a and 21b that are respectively attached to the rotating members 41u and 41r do not come into contact with each other.

A configuration of the switching mechanism 50 is explained with reference to FIGS. 2 to 6.

One end portion of the rotating member supporting shaft 28 is protruded from the exterior case body 6a, and the manipulating lever 18 is attached to the protruded portion.

The switching mechanism 50 is provided with the manipulating lever 18. The engaging portion 51a of the clutch lever 51 is engaged with the engaging groove 33b formed on the fixed gear 33 or disengaged from the engaging groove 33b by manipulating the manipulating lever 18.

The fixed gear 33 that is fixed as described above is rotatably held between the second thrust receiver 27b and the third thrust receiver 27c when the engaging portion 51a is disengaged from the engaging groove 33b. Consequently, the driving force of the motor 24 is not transmitted to the rotating member 41. That is to say, it is impossible to bend the bendable portion 12 by tilting the joystick 17. Such a state is called an angle-free state.

Specifically, the switching mechanism 50 includes the manipulating lever 18, a clutch cam 52, the clutch lever 51, and a clutch lever shaft 53.

The engaging portion 51a and a clutch pin 51b are provided on the clutch lever 51. The clutch lever 51 is integrally secured to a shaft 53a of the clutch lever shaft 53. A cam groove 52a is formed on the clutch cam 52. The clutch cam 52 is integrally secured to the rotating member supporting shaft 28. Further, the manipulating lever 18 is integrally secured to the clutch cam 52.

Hence, the clutch cam 52 is rotated in response to the manipulation of the manipulating lever 18. When the clutch cam 52 is rotated, the clutch pin 51b moves from one end portion side of the cam groove 52a to another end portion side of the cam groove 52b.

Consequently, a position of the clutch lever 51 secured on the clutch lever shaft 53 is changed, and the engaging portion 51a that is provided on the clutch lever 51 is shifted until the engaging portion 51a is disengaged from the engaging groove 33b. Then, the fixed gear 33 that is fixed between the second thrust receiver 27b and the third thrust receiver 27c starts to rotate.

Thus, the manipulation of the manipulating lever 18 allows switching between a state where the engaging portion 51a of the clutch lever 51 is fitted into the engaging groove 33b of the fixed gear 33 and a state where the engaging portion 51a is out of the engaging groove 33b.

In the present embodiment, the clutch lever 51 includes the engaging portion 51a and the fixed gear 33 includes the engaging groove 33b which corresponds to the engaging portion 51a, and form the switching mechanism. When the engaging portion 51a is engaged with the engaging groove 33b, the fixed gear 33 is brought into a fixed state. A structure that brings the fixed gear 33 into the fixed state is not limited to the combination of the engaging portion 51a and the engaging groove 33b. A member having a high friction coefficient, such as an elastic member, may be arranged on the outer circumferential face of the fixed gear 33, and a pressing button (not shown) provided on the clutch lever 51 may be pushed to press the high friction coefficient member to bring the fixed gear 33 into a fixed state. As far as the switching between the engaged state and the disengaged state can be performed through the manipulation of the manipulating lever 18, any structures can be employed.

A bending control of the bendable portion 12 is briefly explained.

Reference character "44" represents a first potentiometer gear. The first potentiometer gear 44 is integrally secured to the rotating external gear 42. Thus, when the rotating external gear 42 is rotated by the movable gear 34, the first potentiometer gear 44 is rotated accordingly.

A second potentiometer gear 45 is engaged with the first potentiometer gear 44. Therefore, the second potentiometer gear 45 is rotated by the rotation of the first potentiometer gear 44. The rotation of the second potentiometer gear 45 is detected by a potentiometer 46. A signal detected by the potentiometer 46 is a signal for calculating an advance and retreat amount of the wires 21a and 21b, and the signal is transmitted through a potentiometer signal cable not shown extending from the potentiometer 46.

The potentiometer signal cable is inserted into the universal cord 7 and extends to the light source connector 7a. Further, the bending controlling connector portion 7c of the light source connector 7a and the bending controlling device 4 are electrically connected by an electric cable 4a. Therefore, a rotated position detecting signal, which shows a rotated position output from the potentiometer 46, is input to a control unit not shown provided at the bending controlling device 4 through the potentiometer signal cable and the electric cable 4a.

Further, an encoder not shown, which is a rotated position detecting unit that serves to detect the rotated position of the motor shaft 24a of the motor 24, is provided at the manipulating portion 6. An encoder signal cable not shown extending from the encoder is inserted into the universal cord 7, and extends to the light source connector 7a. Then, the bending controlling connector portion 7c of the light source connector 7a and the bending controlling device 4 are electrically connected to each other by the electric cable 4a. Consequently, the rotated position detecting signal showing the rotated position of the motor shaft output from the encoder is input to the control unit not shown provided at the bending controlling device 4 through the encoder signal cable and the electric cable 4a.

Furthermore, a bending manipulating command signal showing a tilt angle and a tilt direction of the joystick 17 is output from the joystick 17 provided on the manipulating portion 6. The bending manipulating command signal is output to the control unit of the controlling device 4 by the manipulating portion signal cable extending from the joystick 17. Here, the manipulating portion signal cable is inserted into the universal cord 7, and extends to the light source connector 7a. Then, the bending controlling connector portion 7c of the light source connector 7a and the bending controlling device 4 are electrically connected to each other by the electric cable 4a.

On the other hand, a motor signal cable not shown extends from the motor 24. The motor signal cable is inserted into the universal cord 7, and the motor signal cable extends to the light source connector 7a. Further, the bending controlling connector portion 7c of the light source connector 7a and the bending controlling device 4 are electrically connected to each other by the electric cable 4a. Hence, a motor driving signal output from the control unit not shown provided on the bending controlling device 4 is output to the motor 24 through the electric cable 4a and the motor signal cable.

That is to say, the control unit outputs the motor driving signal to the motor 24 to drive control the motor 24 and thereby bending the bendable portion 12 based on the bending manipulating command signal output from the joystick 17 and based on the rotated position detecting signal output from the encoder and the potentiometer.

An effect of the bending device 20 provided in the manipulating portion 6 of the endoscope 1 configured as described above is explained.

An endoscopy and the like are performed by using the endoscope device 10 while the endoscope 1, the light source 2, the video processor 3, and the bending controlling device 4 are connected as explained with reference to FIG. 1. In such a connection, an operator inserts the distal end portion 11 of the insertion portion 5 towards a target site inside a body cavity while tilting the joystick 17 to bend the bendable portion 12 of the endoscope 1.

When the joystick 17 is tilted, the bending manipulating command signal is output towards the control unit from the joystick 17. Then, the control unit calculates an amount of traction, i.e., a required amount of movement of the bending wire, i.e., an amount of motor rotation, from the bending manipulation command signal. The control unit outputs a motor driving signal corresponding to the calculated value to the motor 24. Then, the motor shaft 24a of the motor 24 is brought into a rotated state.

Then, the sun gear 31 that is disposed at the motor shaft 24a is rotated, and the rotation of the sun gear 31 is transmitted to the planetary gears 32, 32, and 32. Consequently, the planetary gears 32, 32, and 32 start to rotate. At the moment, the fixed gear 33 is not rotated; therefore, the planetary gears 32, 32, and 32 that are engaged with the fixed gear 33 and the movable gear 34 start to rotate and revolve. Since the number of internal teeth 33a of the fixed gear 33 differs from the number of internal teeth 34a of the movable gear 34 by a predetermined number (here, the internal teeth 33a and the internal teeth 34a are the external teeth 32a of the planetary gears 32, 32, and 32), a geared internal tooth 33a becomes shifted with respect to a geared internal tooth 34a. Then, the movable gear 34 rotates at a reduced speed so as to compensate for the shifting.

Then, the rotating external gear 42 having the external teeth 42a that engage with the external teeth 34b of the movable gear 34 is rotated, and the rotating member 41 that is integrally secured to the rotating external gear 42 is rotated. Consequently, the wires 21a and/or 21b are pulled and loosened, and the bendable portion 12 starts to bend.

Here, the first potentiometer gear 44 and the second potentiometer gear 45 are rotated since the rotating external gear 42 is rotated. Since the rotation of the second potentiometer gear 45 is detected by the potentiometer 46, the signal detected by the potentiometer 46 is supplied to the control unit. When the control unit determines that the wires 21a and 21b are advanced or retreated by an amount corresponding to the tilt angle and the tilt direction of the joystick 17, the output of the motor driving signal that is output towards the motor 24 from the control unit is stopped as well as current supplied towards the motor 24 is stopped. Consequently, a desired bending state is obtained.

When an operator determines that the motor 24 might be out of control due to some influences while bending the bendable portion 12 of the endoscope 1, the operator rotates the manipulating lever 18 towards a predetermined direction in order to set the bendable portion 12 in the angle free state. Then, the engaging portion 51a of the clutch lever 51 is disengaged from the engaging groove 33b of the fixed gear 33 in conjunction with the movement of the manipulating lever 18, and the fixed gear 33 that had been fixed starts to rotate. Then, the fixed gear 33 is rotated, and the rotation of the movable gear 34 is stopped. Here, the planetary gears 32, 32, and 32 continue to rotate and revolve because the movable gear 34 obtains load (a) from the wire at the bendable portion 12, and because the fixed gear 33 obtains load (b) from the spring member 27d. Since an amount of the load is a > b, the fixed gear 33 is rotated and the rotation of the movable gear 34 is stopped. That is to say, the bending of the bendable portion 12 is stopped since the transmission of the rotation of the motor shaft 24a to the rotating member 41 is stopped.

When the fixed gear 33 is switched into a rotatable state, the bendable portion 12 is forced to change its state from the bent state into the straightened state due to the rotation of the fixed gear 33 and the movable gear 34 both engaged with the planetary gears 32. However, since the fixed gear 33 is biased by the spring member 27 and the thrust receiver 27c, the fixed gear 33 rotates only at a low speed. Therefore, the state of the bendable portion 12 does not change abruptly, but gradually changes from the bent state to an original, straightened state.

Since the speed reduction mechanism includes the sun gear; the planetary gears; the fixed gear that is the first gear gearing with the planetary gears; and the movable gear that is the second gear having a different number of teeth from the number of teeth of the fixed gear by the predetermined number, the rotational driving force of the sun gear that is rotated by the driving force of the motor can be transmitted by a combination of small gears at a large speed reduction ratio with respect to the movable gear. Consequently, a smaller speed reduction mechanism can be obtained compared with a speed reduction mechanism that includes a large number of gears to have a sufficient speed reduction ratio. Hence, the bending manipulating device in which the speed reduction mechanism is installed can be downsized as well as the manipulating portion can be downsized when the bending manipulating device is provided inside the manipulating portion. Specifically, in a speed reduction mechanism including plural planetary gears, outer diameters of the planetary gears can be decreased since physical strength required for each of the planetary gears is reduced compared to the physical strength required in a speed reduction mechanism including only a single gear. Therefore, the speed reduction mechanism can be further downsized.

Further, the endoscope can be manufactured with low cost because the gears constituting the speed reduction mechanism, i.e., the sun gear, the planetary gears, the fixed gear that is the first gear, and the movable gear that is the second gear, are spur gears that have good workability and do not require high accuracy in gearing.

Furthermore, since the external teeth provided in the movable gear are made to engage with the external teeth of the rotating external gear which is integrally formed with the rotating member, the rotating member supporting shaft is not arranged on an extended line of the motor shaft. Consequently, the bending manipulating device can be downsized, and the manipulating portion can be downsized when the bending manipulating device is provided in the manipulating portion.

Further, since the motor is made flat, the thickness of the manipulating portion that has an aligned structure of the upward and downward bending manipulating device and the leftward and rightward bending manipulating device can be decreased. Further, since the motor has a small outside diameter, the manipulating portion can be downsized by decreasing the distance between the rotating external gear and the rotating member. Consequently, freedom of layout is largely improved by shortening the length of the manipulating portion; adjusting the width of the manipulating portion and miniaturizing the manipulating portion; optimally changing an arrangement (balance) of the manipulating portion; or increasing an internal space inside the manipulating portion.

Furthermore, a spacing between the upward and downward wire and the leftward and rightward wire can be decreased by disposing the movable gear at a motor side with respect to the fixed gear and by arranging the upward and downward rotating member and the leftward and right ward rotating member close to each other while having the separating board therebetween. Consequently, a manipulating portion distal end side can be thinned down as well as the dimension of the bending manipulating device in the direction of the motor shaft can be miniaturized.

As is apparent from the figures, particularly FIG. 2, it is preferred to configure any of the upward and downward bending manipulating device 22 and the leftward and rightward bending manipulating device 23 in such a way that the rotating member can be arranged near an axis that is extended from the central axis of the insertion portion, in order to achieve the downsizing. The traction member (wires 21a and 21b) can be extended coaxially with an axis obtained by extending the central axis of the insertion portion extended from the insertion portion to the manipulating portion. Hence, increasing the spacing between the traction members relating to each of the upward and downward, and leftward and rightward bending manipulation devices can be avoided. This is an advantage that suppresses increasing the shape of the manipulating portion proximal end portion (end portion of insert portion side). Since both of the upward and downward bending manipulating device 22 and the leftward and rightward bending manipulating device 23 have such an advantage, the upward and downward bending manipulating device 22 and the leftward and rightward bending manipulating device 23 are preferably configured to form the rotating member to be arranged at the end portion in the direction of the motor shaft as well as to arrange the rotating member at a position near the axis that is extended from the central axis of the insertion portion.

On the other hand, an object of the present embodiment is to downsize the bending manipulating device. Hence, it is preferred to reduce the dimension thereof in the direction of the motor shaft as much as possible. Further, in the present embodiment, the transmitting gear (rotating external gear 42) is employed as the transmitting mechanism from the speed reduction mechanism to the rotating member, and the rotating member is disposed coaxially with the transmitting gear. Here, it is necessary to dispose the rotating member at a side away from the driving motor with respect to the transmitting gear in order to arrange the rotating member at the end portion in the direction of the motor shaft. However, when the rotating member is protruded in the direction of the motor shaft with respect to the speed reduction mechanism by disposing the rotating member at the position just mentioned, the downsizing of the bending manipulating device relating to the dimension thereof in the direction of the motor shaft might be prevented.

As is apparent from the figures, particularly FIG. 2, the transmitting gear, which is the spur gear engaging with the movable gear, is arranged at a distance away from the driving motor in the direction of the motor shaft substantially identical to the distance from the driving motor to the movable gear in the direction of the motor shaft. Therefore, when the movable gear is arranged at a position away from the driving motor as in a conventional art, that is to say, when the movable gear is arranged at an end portion of the speed reduction mechanism in the direction of the motor shaft, the rotating member that is disposed at a side away from the driving motor with respect to the transmitting gear is to be disposed at a position that is protruded from the speed reduction mechanism with respect to the motor shaft direction. Consequently, the size of the bending manipulating device is reduced.

In order to alleviate the above inconveniences, the movable gear is arranged between the driving motor and the fixed gear in the present embodiment, unlike the general speed reduction mechanism that uses the planetary gears. Then, a space only for the fixed gear is held between a position where the transmitting gear is to be arranged and a position of the end portion of the speed reduction mechanism relating to the direction of the motor shaft, and the movable member is disposed at the space. By employing the configuration described above, the dimension of the bending manipulating device including the movable member in the direction of the motor shaft can be set substantially the same as the dimension of the speed reduction mechanism. Hence, there is an advantage that the bending manipulating device can be downsized with respect to the direction of the motor shaft, compared to a general speed reduction mechanism that uses the planetary gears.

Further, a center of the rotation of the speed reduction mechanism and a center of the rotation of the motor are set to equal to each other, and each of the motors is attached outside of the upward and downward speed reduction mechanism and the leftward and rightward speed reduction mechanism. Consequently, wiring inside the manipulating portion is facilitated as well as exchange of the motor can be performed easily by detaching the exterior case body.

further, by manipulating the manipulating lever provided at the manipulating portion, the fixed gear that is rotatably disposed can be switched to the fixed gear that is disposed literally not to rotate or to the movable gear that rotates. Consequently, when the motor runaway is caused, bending of the bendable portion with an amount more than sufficient can be prevented by switching the disposing state of the fixed gear to the rotating state by manipulating the manipulating lever.

Further, the endoscope is configured so that the manipulation of the manipulating lever achieves the disengagement of the engaging portion from the engaging groove and the removal of the pressing force applied onto the friction member, without the need of special element as a mechanism for switching between the state of the fixed gear and the fixed state to the rotatable state. Consequently, the manipulating portion which is employed to deal with the motor runaway can be downsized and made at low cost.

### INDUSTRIAL APPLICABILITY

As described hereinbefore, the endoscope according to the present invention is useful for an endoscope that bends the bendable portion provided at an insertion portion by driving a driving motor of a bending manipulating device and by pulling and loosening a traction member extending from the bendable portion. Specifically, the endoscope is suitable for applying to an endoscope in which the bending manipulating device is installed in a manipulating portion.

## Claims

1. An endoscope comprising:
an insertion portion (5) that has a bendable portion (12) including plural bending pieces connected with each other; and
a bending manipulating device (22, 23) configured to bend the bendable portion (12) by advancing and retreating a traction member (21a, 21b) extending from the bendable portion (12) and including
a manipulating lever (18) being operated by an operator,
a rotating member (41) rotating with the traction member (21a, 21b) engaged,
a driving motor (24) configured to rotate the rotating member (41), and
a speed reduction mechanism (30) configured to transmit the driving force of the driving motor (24) to the rotating member (41), **characterized in that** the speed reduction mechanism (30) includes a sun gear (31) rotated by the driving force of the motor (24) and having first external teeth, plural planetary gears (32) each having second external teeth engaging with the first external teeth of the sun gear (31), a first fixed gear (33) having first internal teeth engaging with the second external teeth of the plural planetary gears (32), and a second movable gear (34) having second internal teeth engaging with the second external teeth of the plural planetary gears (32), the number of second internal teeth being different from the number of first internal teeth,
wherein the first gear (33) is rotatably disposed and the speed reduction mechanism (30) further comprises a switching mechanism (50) configured to properly switch the first gear (33) from a fixed state where the rotation of the first gear (33) is stopped, to a movable state where the first gear (33) is rotated when the manipulating lever (18) is operated by the operator in conjunction with an operation of the driving motor (24), thereby the first gear (33) starts to rotate and the rotation of the rotatable second gear (34) is stopped while the planetary gears (32) continue to rotate and revolve, and the bending of the bendable portion (12) is stopped.

2. The endoscope according to claim 1, wherein
a motor shaft (24a) of the driving motor (24) is protruded in a direction orthogonal to a traction direction of the traction member (21a, 21b), and the sun gear (31) is provided on the motor shaft (24a).

3. The endoscope according to claim 2, wherein
the second movable gear (34) has third external teeth, and
the rotating member (41) is provided with a transmitting gear (42) which is integrally formed with the rotating member and has fourth external teeth engaging with the third external teeth.

4. The endoscope according to claim 3, wherein
the rotating member (41) is formed coaxially with the transmitting gear (42) and disposed away from the driving motor (24) with respect to the transmitting gear (42), and
the second movable gear (34) is disposed between the driving motor (24) and the first gear (33).

5. The endoscope according to claim 1, wherein
an outside diameter of the second movable gear (34) and an outside diameter of the driving motor (24) are substantially the same.

6. The endoscope according to claim 1, wherein
an outside diameter of the second movable gear (34) is smaller than an outside diameter of the driving motor (24).

## Patentansprüche

1. Endoskop, das umfasst:
einen Einführabschnitt (5), der einen biegbaren Abschnitt (12) mit mehreren miteinander verbundenen Biegeelementen aufweist; und
eine Biegebetätigungseinheit (22, 23), die dazu eingerichtet ist, den biegbaren Abschnitt (12) durch Vor- und Zurückbewegen eines Zugkraftelements (21a, 21b) zu biegen, wobei das Zugkraftelement (21a, 21b) sich von dem biegbaren Abschnitt (12) erstreckt und umfasst:
einen durch einen Benutzer bedienbaren Betätigungshebel (18),
ein Rotationselement (41), das mit dem betätigten Zugkraftelement (21a, 21b) rotiert,
ein Antriebsmotor (24) zum Rotieren des Rotationselements (41), und
eine Geschwindigkeitsdrosseleinrichtung (30) zum Übertragen der Antriebskraft des Antriebsmotors (24) zu dem Rotationselement (41),
**dadurch gekennzeichnet, dass**
die Geschwindigkeitsdrosseleinrichtung (30) ein Sonnenrad (31), das durch die Antriebskraft des Antriebsmotors (24) rotiert wird und erste äußere Zähne aufweist, mehrere Planetenräder (32) mit jeweils zweiten äußeren Zähnen, die mit den ersten äußeren Zähnen des Sonnenrads (31) im Eingriff sind, ein erstes fixiertes Zahnrad (33) mit ersten inneren Zähnen, die mit den zweiten äußeren Zähnen der mehreren Planetenräder (32) im Eingriff sind, und ein zweites bewegliches Zahnrad (34) mit zweiten inneren Zähnen umfasst, die mit den zweiten äußeren Zähnen der mehreren Planetenräder (32) im Eingriff sind, wobei sich die Anzahl der zweiten inneren Zähne von der Anzahl der ersten inneren Zähne unterscheidet, und wobei
das erste Zahnrad (33) rotierbar angeordnet ist und die Geschwindigkeitsdrosseleinrichtung (30) weiterhin einen Schaltmechanismus (50) umfasst, der dazu eingerichtet ist, das erste Zahnrad (33) von einem Haltezustand, in dem die Rotation des ersten Zahnrads (33) angehalten ist, in einen Bewegungszustand, in dem das erste Zahnrad (33) rotiert wird, ordnungsgemäß umzuschalten, wenn der Betätigungshebel (18) durch einen Benutzer im Zusammenhang mit einem Betrieb des Antriebsmotors (24) bedient wird, wodurch das erste Zahnrad (33) zu rotieren beginnt und die Rotation des drehbaren zweiten Zahnrads (34) angehalten wird, während die Planetenräder (32) fortdauernd rotieren und kreisen, und das Biegen des biegbaren Abschnitts (12) beendet wird.

2. Endoskop nach Anspruch 1, wobei
eine Motorwelle (24a) des Antriebsmotors (24) in einer Richtung senkrecht zu einer Zugkraftrichtung des Zugkraftelements (21a, 21b) vorragt, und
das Sonnenrad (31) auf der Motorwelle (24a) bereitgestellt ist.

3. Endoskop nach Anspruch 2, wobei
das zweite bewegliche Zahnrad (34) dritte äußere Zähne umfasst, und
das Rotationselement (41) mit einem Übersetzungszahnrad (42) bereitgestellt ist, das integral mit dem Rotationselement ausgebildet ist und vierte äußere Zähne umfasst, die mit den dritten äußeren Zähnen im Eingriff sind.

4. Endoskop nach Anspruch 3, wobei
das Rotationselement (41) koaxial zu dem Übersetzungszahnrad (42) ausgebildet und mit Bezug auf das Übersetzungszahnrad (42) beabstandet zu dem Antriebsmotor (24) angeordnet ist, und
das zweite bewegliche Zahnrad (34) zwischen dem Antriebsmotor (24) und dem ersten Zahnrad (33) angeordnet ist.

5. Endoskop nach Anspruch 1, wobei
ein Außendurchmesser des zweiten beweglichen Zahnrads (34) und ein Außendurchmesser des Antriebsmotors (24) im Wesentlichen gleich sind.

6. Endoskop nach Anspruch 1, wobei
ein Außendurchmesser des zweiten Zahnrads (34) kleiner ist als ein Außendurchmesser des Antriebsmotors (24).

## Revendications

1. Endoscope comprenant :
une partie d'insertion (5) qui comporte une partie courbable (12) comprenant plusieurs pièces de courbure connectées les unes aux autres ; et
un dispositif (22, 23) de manipulation de courbure configuré pour courber la partie courbable (12) en avançant et en reculant un élément de traction (21a, 21b) s'étendant depuis la partie courbable (12) et comprenant
un levier de manipulation (18) étant actionné par un opérateur,
un élément rotatif (41) tournant avec l'élément de traction (21a, 21b) engagé,
un moteur d'entraînement (24) configuré pour faire tourner l'élément rotatif (41), et
un mécanisme (30) de réduction de vitesse configuré pour transmettre la force d'entraînement du moteur d'entraînement (24) à l'élément rotatif (41), **caractérisé en ce que** le mécanisme (30) de réduction de vitesse comprend un engrenage solaire (31) mis en rotation par la force d'entraînement du moteur (24) et comportant des premières dents externes, plusieurs engrenages planétaires (32) comportant chacun des deuxièmes dents externes s'engageant avec les premières dents externes de l'engrenage solaire (31), un premier engrenage fixe (33) comportant des premières dents internes s'engageant avec les deuxièmes dents externes de la pluralité d'engrenages planétaires (32), et un deuxième engrenage mobile (34) comportant des deuxièmes dents internes s'engageant avec les deuxièmes dents externes de la pluralité d'engrenages planétaires (32), le nombre de deuxièmes dents internes étant différent du nombre de premières dents internes,
dans lequel le premier engrenage (33) est disposé en rotation et le mécanisme (30) de réduction de vitesse comprend en outre un mécanisme de commutation (50) configuré pour commuter correctement le premier engrenage (33) d'un état fixe dans lequel la rotation du premier engrenage (33) est arrêtée, à un état mobile dans lequel le premier engrenage (33) est mis en rotation lorsque le levier de manipulation (18) est actionné par l'opérateur en liaison avec une opération du moteur d'entraînement (24), de ce fait l'engrenage (33) commence à tourner et la rotation du deuxième engrenage (34) rotatif est arrêtée alors que les engrenages planétaires (32) continuent de tourner et de se mettre en prise, et la courbure de la partie courbable (12) est arrêtée.

2. Endoscope selon la revendication 1, dans lequel
un arbre de moteur (24a) du moteur d'entraînement (24) est projeté dans une direction orthogonale à une direction de traction de l'élément de traction (21a, 21b), et l'engrenage solaire (31) est prévu sur l'arbre de moteur (24a).

3. Endoscope selon la revendication 2, dans lequel
le deuxième engrenage mobile (34) comporte des troisièmes dents externes, et
l'élément rotatif (41) est muni d'un engrenage de transmission (42) qui est solidairement formé avec l'élément rotatif et comporte des quatrièmes dents externes s'engageant avec les troisièmes dents externes.

4. Endoscope selon la revendication 3, dans lequel
l'élément rotatif (41) est formé coaxialement avec l'engrenage de transmission (42) et disposé éloigné du moteur d'entraînement (24) par rapport à l'engrenage de transmission (42), et
le deuxième engrenage mobile (34) est disposé entre le moteur d'entraînement (24) et le premier engrenage (33).

5. Endoscope selon la revendication 1, dans lequel
un diamètre extérieur du deuxième engrenage mobile (34) et un diamètre extérieur du moteur d'entraînement (24) sont sensiblement les mêmes.

6. Endoscope selon la revendication 1, dans lequel
un diamètre extérieur du deuxième engrenage mobile (34) est plus petit qu'un diamètre extérieur du moteur d'entraînement (24).
